# EUROPÄISCHE PATENTANMELDUNG

(11) **EP 3 598 949 A1**
(43) Veröffentlichungstag der Anmeldung: **29.01.2020**
(21) Anmeldenummer: 18186081.8
(22) Anmeldetag: 27.07.2018
(51) Int. Cl.: A61B 6/03, A61B 6/06, G21K 1/02, A61B 6/00, G01N 23/046, H01J 35/14, H01J 35/24, H01J 35/30, G01V 5/00, G21K 1/10

(54) **COMPUTERTOMOGRAPHIEGERÄT MIT LAMELLENFORMFILTER UND SPRINGFOKUS-RÖNTGENQUELLE**

(71) Anmelder: Siemens Healthcare GmbH, 91052 Erlangen (DE)
(72) Erfinder: Huck, Sascha Manuel, 91088 Bubenreuth (DE); Stierstorfer, Karl, 91052 Erlangen (DE)

(57) **Zusammenfassung**

Die Erfindung betrifft ein Computertomographiegerät, aufweisen:
- eine Röntgenquelle und einen Röntgendetektor, der mit der Röntgenquelle zusammenwirkt, wobei die Röntgenquelle und der Röntgendetektor um eine Rotationsachse, die sich zwischen der Röntgenquelle und dem Röntgendetektor befindet, drehbar angeordnet sind,
- einen Lamellenformfilter, der zwischen der Röntgenquelle und der Rotationsachse angeordnet ist, wobei der Lamellenformfilter eine erste Mehrzahl von ersten Lamellen aufweist, welche auf eine erste Fokuslinie fokussiert sind,
- wobei die Röntgenquelle dazu ausgebildet ist, Röntgenstrahlung in einem ersten Betriebszustand der Röntgenquelle an einem ersten Fokuspunkt zu erzeugen und in einem zweiten Betriebszustand der Röntgenquelle an einem zweiten Fokuspunkt zu erzeugen.

## Beschreibung

Die Erfindung betrifft ein Computertomographiegerät mit Lamellenformfilter. Die Erfindung betrifft ferner ein Verfahren zum Erzeugen eines medizinischen Bildes.

Ein Lamellenformfilter ermöglicht eine variable und situationsspezifische Einstellung einer Intensität einer Röntgenstrahlung. Für diesen Zweck werden Lamellen des Lamellenformfilters, welche die Röntgenstrahlung absorbieren, präzise auf eine gemeinsame oder auf unterschiedliche Fokuslinien ausgerichtet. Somit können die Lamellen auf einen Fokuspunkt, an dem die Röntgenstrahlen emittiert werden, fokussiert oder in Bezug auf den Fokuspunkt defokussiert werden. In dem Fall, dass sich der Fokuspunkt auf einer Fokuslinie befindet, wird die Röntgenstrahlung durch diejenigen Lamellen, die auf diese Fokuslinie ausgerichtet sind, nur unwesentlich abgeschwächt. Die Abschwächung durch diese Lamellen ist deutlich stärker, wenn der Fokuspunkt sich nicht auf der entsprechenden Fokuslinie befindet. Die Ausrichtung der Lamellen kann insbesondere in Abhängigkeit von einem Drehwinkel der Drehung der Röntgenquelle um eine Rotationsachse erfolgen.

Bei einem Computertomographiegerät rotiert die Röntgenquelle mit einer relativ hohen Geschwindigkeit, beispielsweise ca. 4 Hz. Daher ist eine sehr schnelle Anpassung der Lamellenausrichtung an die gegebene Situation, insbesondere an den Drehwinkel, erforderlich. Insbesondere kann es erforderlich sein, den Lamellenformfilter und/oder die Lamellen des Lamellenformfilters relativ schnell derart auszurichten, dass eine Fokuslinie, auf welche die Lamellen fokussiert sind, eine vorgegebene Lage relativ zu einem Fokuspunkt einnimmt.

Dabei sind die Anforderungen an Reproduzierbarkeit und Genauigkeit der Mechanik, welche die Lamellen ausrichtet, sehr hoch. Kleine Abweichungen in der Ausrichtung der Lamellen können die Intensität der gefilterten Röntgenstrahlung stark beeinflussen. Die Bewegung des Lamellenformfilters und/oder der Lamellen relativ zu der Röntgenquelle beträgt dabei beispielsweise bis zu einigen Grad. Eine Abhängigkeit der Intensität der gefilterten Röntgenstrahlung von der Lamellenausrichtung kann beispielsweise durch eine Kalibration und/oder durch Referenzmessungen ermittelt werden.

EP 3217408 A2 offenbart ein Fokussierungsmodul für einen Formfilter zum Einstellen einer räumlichen Intensitätsverteilung eines Röntgenstrahls.

US 2018/0033514 A1 offenbart ein Verfahren zum Einstellen einer räumlichen Intensitätsverteilung eines Röntgenstrahls, wobei ein Röntgenstrahl erzeugt wird und ein Strahlengang des Röntgenstrahls in einer Ausbreitungsrichtung durch einen Formfilter mit einer Mehrzahl an Lamellenblechen geführt wird.

US 2017/0011815 A1 offenbart eine Röntgenfilteranordnung, umfassend eine Mehrzahl von Röntgenstrahldämpfungsschichten, die in einem Stapel angeordnet sind.

Die Erfindung hat die Aufgabe, eine verbesserte Einstellung einer Intensität einer Röntgenstrahlung unter Verwendung eines Lamellenformfilters zu ermöglichen. Jeder Gegenstand eines unabhängigen Anspruchs löst diese Aufgabe. In den abhängigen Ansprüchen sind weitere vorteilhafte Aspekte der Erfindung berücksichtigt.

Die Erfindung betrifft ein Computertomographiegerät, aufweisend:
- eine Röntgenquelle und einen Röntgendetektor, der mit der Röntgenquelle zusammenwirkt, wobei die Röntgenquelle und der Röntgendetektor um eine Rotationsachse, die sich zwischen der Röntgenquelle und dem Röntgendetektor befindet, drehbar angeordnet sind,
- einen Lamellenformfilter, der zwischen der Röntgenquelle und der Rotationsachse angeordnet ist, wobei der Lamellenformfilter eine erste Mehrzahl von ersten Lamellen aufweist, welche auf eine erste Fokuslinie fokussiert sind,
- wobei die Röntgenquelle dazu ausgebildet ist, Röntgenstrahlung in einem ersten Betriebszustand der Röntgenquelle an einem ersten Fokuspunkt zu erzeugen und in einem zweiten Betriebszustand der Röntgenquelle an einem zweiten Fokuspunkt zu erzeugen.

Eine Ausführungsform sieht vor, dass der erste Fokuspunkt und der zweite Fokuspunkt relativ zueinander zumindest in Bezug auf eine Axialrichtung versetzt sind, welche parallel zu der Rotationsachse ist. Die Axialrichtung kann insbesondere entlang einer Tiefe eines Fächerstrahls der Röntgenstrahlung ausgerichtet sein.

Eine Ausführungsform sieht vor, dass der erste Fokuspunkt und der zweite Fokuspunkt relativ zueinander zumindest in Bezug auf eine Radialrichtung versetzt sind, welche die Rotationsachse schneidet und welche senkrecht zu der Rotationsachse ist. Die Radialrichtung kann insbesondere entlang eines Zentralstrahls eines Fächerstrahls der Röntgenstrahlung ausgerichtet sein.

Eine Ausführungsform sieht vor, dass der erste Fokuspunkt und der zweite Fokuspunkt relativ zueinander zumindest in Bezug auf eine Umfangsrichtung versetzt sind, welche im Wesentlichen senkrecht zu einer Ebene ist, in welcher sich die Rotationsachse befindet und in welcher sich ferner der erste Fokuspunkt oder der zweite Fokuspunkt befindet. Die Umfangsrichtung kann insbesondere entlang einer Breite eines Fächerstrahls der Röntgenstrahlung ausgerichtet sein. Insbesondere kann vorgesehen sein, dass eine Gerade, welche durch den ersten Fokuspunkt und durch den zweiten Fokuspunkt geht, im Wesentlichen senkrecht zu einer Ebene ist, in welcher sich die Rotationsachse befindet und in welcher sich ferner der erste Fokuspunkt oder der zweite Fokuspunkt befindet.

Eine Ausführungsform sieht vor, dass sich die Rotationsachse, der erste Fokuspunkt und der zweite Fokuspunkt in einer Ebene befinden. Eine Ausführungsform sieht vor, dass sich der erste Fokuspunkt auf der ersten Fokuslinie befindet und dass sich der zweite Fokuspunkt nicht auf der ersten Fokuslinie befindet.

Eine Ausführungsform sieht vor, dass der Lamellenformfilter eine zweite Mehrzahl von zweiten Lamellen aufweist, welche auf eine zweite Fokuslinie fokussiert sind und dass sich der zweite Fokuspunkt auf der zweiten Fokuslinie befindet. Ausführungsformen können auch in Bezug auf die zweiten Lamellen durch Merkmale weitergebildet sein, die im Zusammenhang mit den ersten Lamellen offenbart sind. Insbesondere kann vorgesehen sein, dass es zusätzlich zu der ersten Fokuslinie und der zweiten Fokuslinie weitere Fokuslinien gibt, auf welche jeweils eine weitere Mehrzahl von Lamellen des Lamellenformfilters fokussiert ist.

Eine Ausführungsform sieht vor, dass die zweite Fokuslinie parallel zu der ersten Fokuslinie ist. Eine Ausführungsform sieht vor, dass die erste Fokuslinie im Wesentlichen parallel insbesondere parallel, zu der Rotationsachse ist und/oder die zweite Fokuslinie im Wesentlichen parallel, insbesondere parallel, zu der Rotationsachse ist.

Eine Ausführungsform sieht vor, dass die erste Fokuslinie und/oder die zweite Fokuslinie in einer Ebene liegt, die senkrecht zu der Rotationsachse ist. Insbesondere kann der Lamellenformfilter eine Lamelle aufweisen, welche in einem zentralen Bereich des Lamellenformfilters angeordnet ist und welche im Wesentlichen in einer zu der Rotationsachse senkrechten Ebene liegt.

Eine Ausführungsform sieht vor, dass die Röntgenquelle eine Kathode, eine Anode und eine Ablenkvorrichtung aufweist, wobei die Ablenkvorrichtung dazu ausgebildet ist, eine Ablenkung eines Elektronenstrahls, der von der Kathode ausgeht, derart einzustellen, dass der Elektronenstrahl in dem ersten Betriebszustand der Röntgenquelle in dem ersten Fokuspunkt auf die Anode trifft und in dem zweiten Betriebszustand der Röntgenquelle in dem zweiten Fokuspunkt auf die Anode trifft.

Insbesondere kann die Anode in Form einer Anodenscheibe ausgebildet sein. Insbesondere kann die Ablenkung des Elektronenstrahls eine elektromagnetische Ablenkung des Elektronenstrahls sein. Die Ablenkvorrichtung kann beispielsweise einen oder mehrere Elektromagneten zur elektromagnetischen Ablenkung des Elektronenstrahls aufweisen.

Eine Ausführungsform sieht ein Computertomographiegerät vor, ferner aufweisend:
- eine Positioniereinheit,
- wobei die Positioniereinheit zum Positionieren des Lamellenformfilters relativ zu der Röntgenquelle ausgebildet ist und der Lamellenformfilter mittels der Positioniereinheit derart relativ zu der Röntgenquelle positionierbar ist, dass durch eine Positionierung des Lamellenformfilters relativ zu der Röntgenquelle eine Lage der ersten Fokuslinie relativ zu dem ersten Fokuspunkt geändert werden kann, und/oder
- wobei die Positioniereinheit zum Positionieren der ersten Lamellen des Lamellenformfilters relativ zueinander ausgebildet ist und die ersten Lamellen des Lamellenformfilters mittels der Positioniereinheit derart relativ zueinander positionierbar sind, dass durch eine Positionierung der ersten Lamellen des Lamellenformfilters relativ zueinander eine Lage der ersten Fokuslinie relativ zu dem ersten Fokuspunkt geändert werden kann.

Die Positioniereinheit kann alternativ oder zusätzlich dazu zum Positionieren der zweiten Lamellen des Lamellenformfilters relativ zueinander ausgebildet sein. Insbesondere können die zweiten Lamellen des Lamellenformfilters mittels der Positioniereinheit derart relativ zueinander positionierbar sein, dass durch eine Positionierung der zweiten Lamellen des Lamellenformfilters relativ zueinander eine Lage der zweiten Fokuslinie relativ zu dem ersten Fokuspunkt und/oder relativ zu dem zweiten Fokuspunkt geändert werden kann.

Dadurch wird ein zusätzlicher Freiheitsgrad bereitgestellt, um zusätzliche Variationen der transmittierten Intensität der Röntgenstrahlung zu ermöglichen. Beispielsweise kann vorgesehen sein, dass die Positionierung des Lamellenformfilters relativ zu der Röntgenquelle und/oder die Positionierung der ersten Lamellen relativ zueinander in Abhängigkeit von einem Drehwinkel der Drehung der Röntgenquelle und des Röntgendetektors um die Rotationsachse erfolgt.

Die Positioniereinheit kann insbesondere einen oder mehrere Aktoren für die Positionierung des Lamellenformfilters relativ zu der Röntgenquelle aufweisen. Insbesondere kann für jede Schwenkachse, um welche der Lamellenformfilter schwenkbar gelagert ist, jeweils ein Aktor vorgesehen sein, der eine Schwenkbewegung des Lamellenformfilters um die Schwenkachse antreiben kann. Der Lamellenformfilter kann insbesondere um eine oder mehrere Schwenkachsen schwenkbar gelagert sein, welche beispielsweise einen zentralen Bereich des Lamellenformfilters durchdringen können.

Der Lamellenformfilter kann beispielsweise um zwei Schwenkachsen schwenkbar gelagert sein, wobei eine Schwenkachse parallel zu der Rotationsachse und/oder zu der ersten Fokuslinie ist und die andere Schwenkachse senkrecht zu der ersten Schwenkachse, insbesondere im Wesentlichen parallel zu der Umfangsrichtung, ist. Ein Aktor für die Positionierung des Lamellenformfilters relativ zu der Röntgenquelle kann beispielsweise ein Schrittmotor oder ein Piezomotor sein.

Die Positioniereinheit kann insbesondere einen oder mehrere Aktoren für die Positionierung der Lamellen relativ zueinander aufweisen. Insbesondere kann für jede Lamelle zumindest ein Aktor vorgesehen sein, welcher die Ausrichtung dieser Lamelle schnell und präzise ändern kann. Insbesondere können Aktionen der Aktoren zur Ausrichtung der Lamellen mit der Drehung der Röntgenquelle und des Röntgendetektors um die Rotationsachse synchronisiert werden. Ein Aktor für die Positionierung der Lamellen relativ zueinander kann beispielsweise ein Piezomotor oder ein Aktor auf Basis eines elektroaktiven Polymers sein.

Die Erfindung betrifft ferner ein Verfahren zum Erzeugen eines medizinischen Bildes, wobei das Verfahren die folgenden Schritte umfasst:
- Drehen einer Röntgenquelle und eines Röntgendetektors, der mit der Röntgenquelle zusammenwirkt, um eine Rotationsachse, die sich zwischen der Röntgenquelle und dem Röntgendetektor befindet,
- Erzeugen einer Röntgenstrahlung mittels einer Röntgenquelle an einem ersten Fokuspunkt, während die Röntgenquelle in einem ersten Betriebszustand der Röntgenquelle ist,
- Filtern der Röntgenstrahlung, die an dem ersten Fokuspunkt erzeugt wurde, mittels eines Lamellenformfilters, der zwischen der Röntgenquelle und der Rotationsachse angeordnet ist, wobei der Lamellenformfilter eine erste Mehrzahl von ersten Lamellen aufweist, welche auf eine erste Fokuslinie fokussiert sind,
- Erfassen von ersten Projektionsdaten mittels des Röntgendetektors basierend auf der Röntgenstrahlung, welche an dem ersten Fokuspunkt erzeugt wurde und mittels des Lamellenformfilters gefiltert wurde,
- Übergang von dem ersten Betriebszustand der Röntgenquelle in einen zweiten Betriebszustand der Röntgenquelle,
- Erzeugen der Röntgenstrahlung mittels der Röntgenquelle an einem zweiten Fokuspunkt, während die Röntgenquelle in dem zweiten Betriebszustand der Röntgenquelle ist,
- Filtern der Röntgenstrahlung, die an dem zweiten Fokuspunkt erzeugt wurde, mittels des Lamellenformfilters, der zwischen der Röntgenquelle und der Rotationsachse angeordnet ist, wobei der Lamellenformfilter eine erste Mehrzahl von ersten Lamellen aufweist, welche auf eine erste Fokuslinie fokussiert sind,
- Erfassen von zweiten Projektionsdaten mittels des Röntgendetektors basierend auf der Röntgenstrahlung, welche an dem zweiten Fokuspunkt erzeugt wurde und mittels des Lamellenformfilters gefiltert wurde,
- Erzeugen eines medizinischen Bildes basierend auf den ersten Projektionsdaten und den zweiten Projektionsdaten.

Eine Ausführungsform sieht ein Verfahren vor, das ferner den folgenden Schritt umfasst:
- Übergang von einem ersten Betriebszustand des Lamellenformfilters in einen zweiten Betriebszustand des Lamellenformfilters derart, dass sich dabei die Lage der ersten Fokuslinie relativ zu dem ersten Fokuspunkt ändert,
- wobei die Röntgenstrahlung mittels der Röntgenquelle an dem ersten Fokuspunkt erzeugt wird, während die Röntgenquelle in dem ersten Betriebszustand der Röntgenquelle und der Lamellenformfilter in dem ersten Betriebszustand des Lamellenformfilters ist,
- wobei die Röntgenstrahlung mittels der Röntgenquelle an dem zweiten Fokuspunkt erzeugt wird, während die Röntgenquelle in dem zweiten Betriebszustand der Röntgenquelle und der Lamellenformfilter in dem zweiten Betriebszustand des Lamellenformfilters ist.

Eine Ausführungsform sieht vor, dass der Übergang von dem ersten Betriebszustand der Röntgenquelle in den zweiten Betriebszustand der Röntgenquelle und/oder der Übergang von dem ersten Betriebszustand des Lamellenformfilters in den zweiten Betriebszustand des Lamellenformfilters in Abhängigkeit von einem Drehwinkel der Drehung der Röntgenquelle und des Röntgendetektors um die Rotationsachse erfolgt.

Insbesondere können der erste Fokuspunkt und der zweite Fokuspunkt einen unterschiedlichen Grad der Defokussierung in Bezug auf die erste Fokuslinie und/oder in Bezug auf die zweite Fokuslinie aufweisen. Somit hat nach der Filterung mittels des Lamellenformfilters die Röntgenstrahlung, die an dem ersten Fokuspunkt erzeugt wurde, eine andere Intensität als die Röntgenstrahlung, die an dem zweiten Fokuspunkt erzeugt wurde. Die erste Fokuslinie kann insbesondere gerade sein. Die zweite Fokuslinie kann insbesondere gerade sein. Insbesondere kann vorgesehen sein, dass sich die erste Fokuslinie und/oder die zweite Fokuslinie in einem Bereich der Röntgenquelle befindet. Eine Lamelle ist insbesondere dann auf eine gegebene Fokuslinie fokussiert, wenn sich die Lamelle in einer Ebene befindet, welche die gegebene Fokuslinie aufweist. Lamellen können insbesondere aus einem die Röntgenstrahlung absorbierenden Material, beispielsweise einem Metall, hergestellt sein und/oder jeweils als dünne flächige Teile, beispielsweise in Form eines Blechs, ausgebildet sein.

Gemäß einer Ausführungsform beträgt ein Abstand zwischen dem ersten Fokuspunkt und dem zweiten Fokuspunkt mindestens 0,1 Millimeter, bevorzugt mindestens 0,3 Millimeter, insbesondere bevorzugt mindestens 1 Millimeter. Gemäß einer Ausführungsform beträgt ein Abstand zwischen dem ersten Fokuspunkt und dem zweiten Fokuspunkt in Bezug auf eine Axialrichtung, welche parallel zu der Rotationsachse ist, mindestens 0,05 Millimeter, bevorzugt mindestens 0,1 Millimeter, insbesondere bevorzugt mindestens 0,15 Millimeter.

Insbesondere kann vorgesehen sein, dass eine Gerade, welche durch den ersten Fokuspunkt und durch den zweiten Fokuspunkt geht, nicht parallel zu der ersten Fokuslinie ist. Insbesondere kann vorgesehen sein, dass sich der zweite Fokuspunkt nicht auf der ersten Fokuslinie befindet und/oder dass sich der erste Fokuspunkt nicht auf der zweiten Fokuslinie befindet. Insbesondere kann vorgesehen sein, dass der erste Fokuspunkt und/oder der zweite Fokuspunkt relativ zu der Röntgenquelle definiert ist. Insbesondere kann vorgesehen sein, dass die erste Fokuslinie und/oder die zweite Fokuslinie relativ zu dem Lamellenformfilter definiert ist.

Insbesondere kann vorgesehen sein, dass die Röntgenquelle zusätzlich zu dem ersten Betriebszustand und dem zweiten Betriebszustand weitere Betriebszustände einnehmen kann und/oder dass die Röntgenquelle zusätzlich zu dem ersten Fokuspunkt und dem zweiten Fokuspunkt an weiteren Fokuspunkten Röntgenstrahlung erzeugen kann.

Gemäß einer Ausführungsform ist vorgesehen, dass die Röntgenquelle dazu ausgebildet ist, Röntgenstrahlung an allen Fokuspunkten einer Mehrzahl von Fokuspunkten, die zusammen einen Pfad von dem ersten Fokuspunkt zu dem zweiten Fokuspunkt bilden, zu erzeugen. Insbesondere kann sich während des Übergangs von dem ersten Betriebszustand der Röntgenquelle in den zweiten Betriebszustand der Röntgenquelle der ein Fokuspunkt, an dem der Elektronenstrahl auf die Anode trifft und an dem Röntgenstrahlung erzeugt wird, kontinuierlich entlang eines Pfades von dem ersten Fokuspunkt zu dem zweiten Fokuspunkt bewegt. Unter einem Fokuspunkt kann insbesondere ein Punkt verstanden werden, an dem ein Zentralstrahl des Elektronenstrahls auf die Anode trifft.

Gemäß einer Ausführungsform ist vorgesehen, dass während des Übergangs von dem ersten Betriebszustand der Röntgenquelle in den zweiten Betriebszustand der Röntgenquelle keine Röntgenstrahlung erzeugt wird.

Eine elektromagnetische Ablenkung des Elektronenstrahls und die damit verbundene Bewegung des Fokuspunktes kann im Allgemeinen schneller, präziser und mit besserer Reproduzierbarkeit erfolgen als mechanische Vorrichtungen die Lamellen verstellen können. Auf diese Weise könnten die Abweichungen reduziert und die Qualität der Intensitätsmodulation gesteigert werden. Ferner kann auf eine mechanische Positioniereinheit zur Bewegung des Lamellenformfilters verzichtet werden, wodurch die damit verbundenen Kosten und potentielle Fehlerquellen vermieden werden können.

Der Lamellenformfilter kann feststehend im Strahlengang, beispielsweise in einem Blendenkasten zusammen mit Blenden zur Kollimation der Röntgenstrahlung, angeordnet werden. Auf diese Weise kann der Lamellenformfilter vor äußeren Einflüssen besser geschützt werden. Insbesondere können damit die auf den Lamellenformfilter bei der Drehung wirkenden Zentripetalkräfte besser aufgenommen werden.

Somit kann die Intensität der Röntgenstrahlung moduliert werden, ohne dass der Lamellenformfilter relativ zu der Röntgenquelle bewegt werden muss. Vorrichtungen zur kontrollierten Ablenkung eines Elektronenstrahls in einer Röntgenquelle, um Röntgenstrahlung an verschiedenen Fokuspunkten zu erzeugen, sind bereits bekannt.

US7313214B2 offenbart ein Computertomographiegerät mit mindestens einer Röntgenröhre, die einen relativ zur Röntgenröhre ortsveränderlichen Fokus (Springfokus) aufweist.

Insbesondere kann die Einstellung der Intensität der Röntgenstrahlung basierend auf einem ortsveränderlichen Fokus mit einem geringeren technischen Aufwand realisiert werden als die Einstellung der Intensität der Röntgenstrahlung basierend auf einer Bewegung der Lamellen relativ zu der Röntgenquelle. Verfahren zur Kalibrierung einer Vorrichtungen zur kontrollierten Ablenkung eines Elektronenstrahls, die dem Fachmann möglicherweise im Zusammenhang mit einem Springfokus (Flying Focus, Flying Focal Spot) bekannt sind, können bei der Kalibrierung der Ablenkvorrichtung berücksichtigt werden, damit sich der erste Fokuspunkt und der zweiten Fokuspunkt jeweils an einer dafür geeigneten Position der Anode befinden.

Im Rahmen der Erfindung können Merkmale, welche in Bezug auf unterschiedliche Ausführungsformen der Erfindung und/oder unterschiedliche Anspruchskategorien (Verfahren, Verwendung, Vorrichtung, System, Anordnung usw.) beschrieben sind, zu weiteren Ausführungsformen der Erfindung kombiniert werden. Beispielsweise kann ein Anspruch, der eine Vorrichtung betrifft, auch mit Merkmalen, die im Zusammenhang mit einem Verfahren beschrieben oder beansprucht sind, weitergebildet werden und umgekehrt. Funktionale Merkmale eines Verfahrens können dabei durch entsprechend ausgebildete gegenständliche Komponenten ausgeführt werden. Neben den in dieser Anmeldung ausdrücklich beschriebenen Ausführungsformen der Erfindung sind vielfältige weitere Ausführungsformen der Erfindung denkbar, zu denen der Fachmann gelangen kann, ohne den Bereich der Erfindung zu verlassen, der durch die Ansprüche vorgegeben ist.

Die Verwendung der unbestimmten Artikel "ein" bzw. "eine" schließt nicht aus, dass das betroffene Merkmal auch mehrfach vorhanden sein kann. Die Verwendung des Ausdrucks "aufweisen" schließt nicht aus, dass die mittels des Ausdrucks "aufweisen" verknüpften Begriffe identisch sein können. Beispielsweise weist das Computertomographiegerät das Computertomographiegerät auf. Die Verwendung des Ausdrucks "Einheit" schließt nicht aus, dass der Gegenstand, auf den sich der Ausdruck "Einheit" bezieht, mehrere Komponenten aufweisen kann, die räumlich voneinander separiert sind.

Im Folgenden wird die Erfindung anhand von Ausführungsbeispielen unter Hinweis auf die beigefügten Figuren erläutert. Die Darstellung in den Figuren ist schematisch, stark vereinfacht und nicht zwingend maßstabsgetreu.

Es zeigen:
die Fig. 1 eine Röntgenquelle in einem ersten Betriebszustand und einen Lamellenformfilter,
die Fig. 2 eine Röntgenquelle in einem zweiten Betriebszustand und einen Lamellenformfilter,
die Fig. 3 eine Röntgenquelle in einem ersten Betriebszustand und einen Lamellenformfilter mit einer ersten Fokuslinie und einer zweiten Fokuslinie,
die Fig. 4 eine Röntgenquelle in einem zweiten Betriebszustand und einen Lamellenformfilter mit einer ersten Fokuslinie und einer zweiten Fokuslinie,
die Fig. 5 eine Röntgenquelle und einen Lamellenformfilter, wobei der erste Fokuspunkt und der zweite Fokuspunkt relativ zueinander entlang einer Breite des Fächerstrahls versetzt sind,
die Fig. 6 eine Röntgenquelle und einen Lamellenformfilter, wobei der Lamellenformfilter relativ zu der Röntgenquelle positionierbar ist,
die Fig. 7 eine Röntgenquelle in einem ersten Betriebszustand und einen Lamellenformfilter, wobei die erste Fokuslinie in einer Ebene liegt, die senkrecht zu der Rotationsachse ist,
die Fig. 8 eine Röntgenquelle in einem zweiten Betriebszustand und einen Lamellenformfilter, wobei die erste Fokuslinie in einer Ebene liegt, die senkrecht zu der Rotationsachse ist,
die Fig. 9 eine Röntgenquelle und einen Lamellenformfilter, wobei die erste Fokuslinie in einer Ebene liegt, die senkrecht zu der Rotationsachse ist, wobei der Lamellenformfilter relativ zu der Röntgenquelle positionierbar ist,
die Fig. 10 ein Computertomographiegerät mit einer Röntgenquelle und einem Lamellenformfilter,
die Fig. 11 ein Ablaufdiagramm eines Verfahrens zum Erzeugen eines medizinischen Bildes, und
die Fig. 12 ein Ablaufdiagramm eines Verfahrens zum Erzeugen eines medizinischen Bildes gemäß einem weiterem Ausführungsbeispiel.

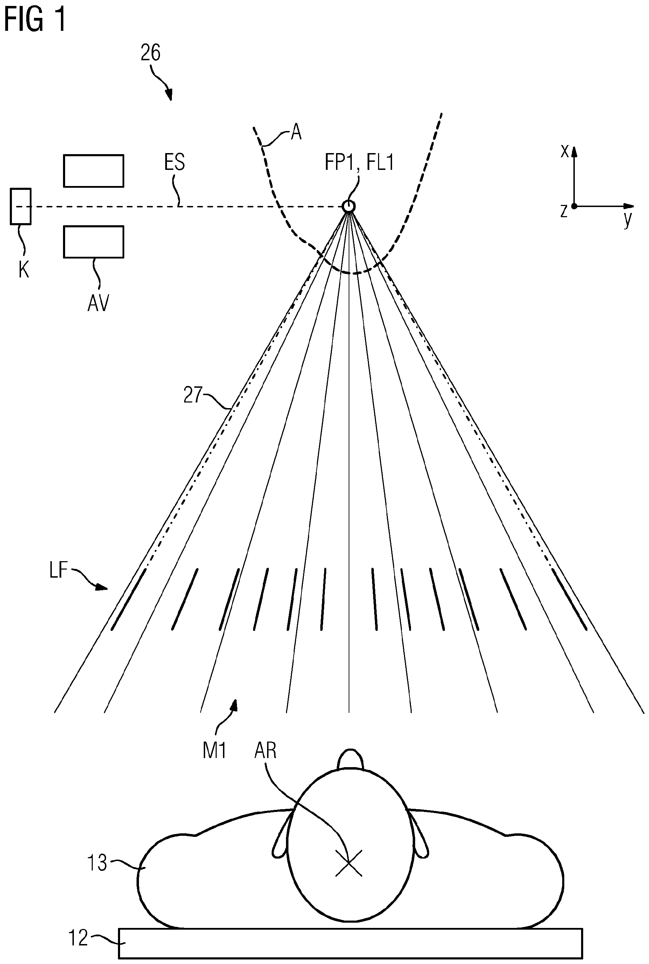

Die Fig. 1 zeigt eine Röntgenquelle 26 in einem ersten Betriebszustand und einen Lamellenformfilter LF. Die Röntgenquelle 26 wirkt mit einem Röntgendetektor 28 zusammen. Die Röntgenquelle 26 und der Röntgendetektor 28 sind um eine Rotationsachse AR, die sich zwischen der Röntgenquelle 26 und dem Röntgendetektor 28 befindet, drehbar angeordnet. Der Lamellenformfilter LF ist zwischen der Röntgenquelle 26 und der Rotationsachse AR angeordnet und weist eine erste Mehrzahl von ersten Lamellen M1 auf, welche auf eine erste Fokuslinie FL1 fokussiert sind. Die Röntgenquelle 26 ist dazu ausgebildet, Röntgenstrahlung 27 in einem ersten Betriebszustand der Röntgenquelle 26 an einem ersten Fokuspunkt FP1 zu erzeugen und in einem zweiten Betriebszustand der Röntgenquelle 26 an einem zweiten Fokuspunkt FP2 zu erzeugen.

Die Röntgenquelle 26 weist eine Kathode K, eine Anode A und eine Ablenkvorrichtung AV auf, wobei die Ablenkvorrichtung AV dazu ausgebildet ist, eine Ablenkung eines Elektronenstrahls ES, der von der Kathode K ausgeht, derart einzustellen, dass der Elektronenstrahl ES in dem ersten Betriebszustand der Röntgenquelle 26 in dem ersten Fokuspunkt FP1 auf die Anode A trifft und in dem zweiten Betriebszustand der Röntgenquelle 26 in dem zweiten Fokuspunkt FP2 auf die Anode A trifft.

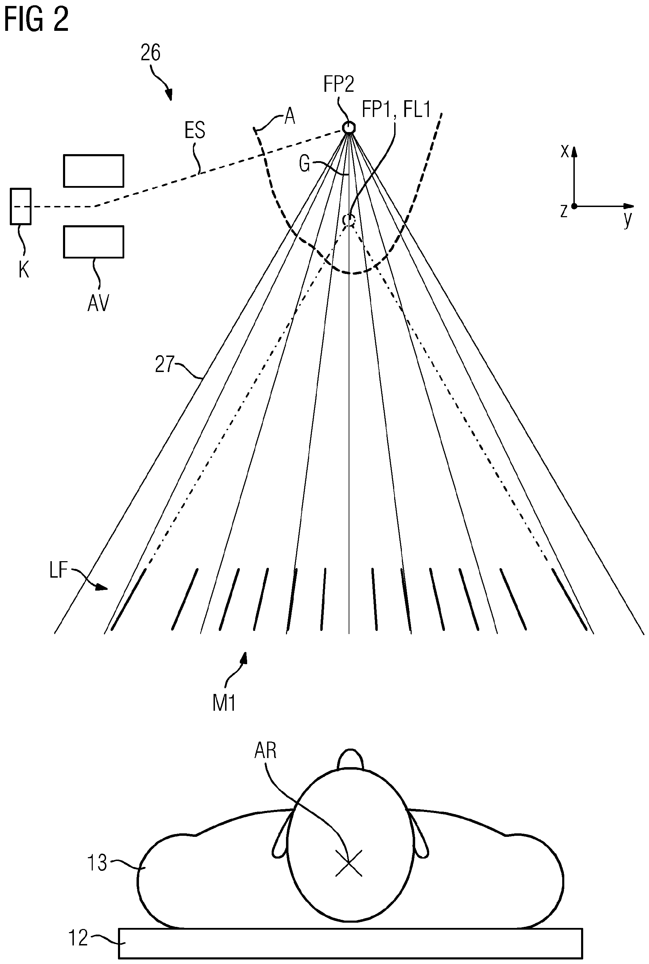

Die Fig. 2 zeigt die Röntgenquelle 26 und den Lamellenformfilter LF gemäß dem in der Fig. 1 gezeigten Ausführungsbeispiel, wobei die Röntgenquelle 26 in einem zweiten Betriebszustand ist. Der erste Fokuspunkt FP1 und der zweite Fokuspunkt FP2 sind relativ zueinander in Bezug auf eine Radialrichtung x versetzt, welche die Rotationsachse AR schneidet und welche senkrecht zu der Rotationsachse AR ist. Der erste Fokuspunkt FP1 befindet sich auf der ersten Fokuslinie FL1. Der zweite Fokuspunkt FP2 befindet sich nicht auf der ersten Fokuslinie FL1. Die Rotationsachse AR, der erste Fokuspunkt FP1 und der zweite Fokuspunkt FP2 befinden sich in einer Ebene.

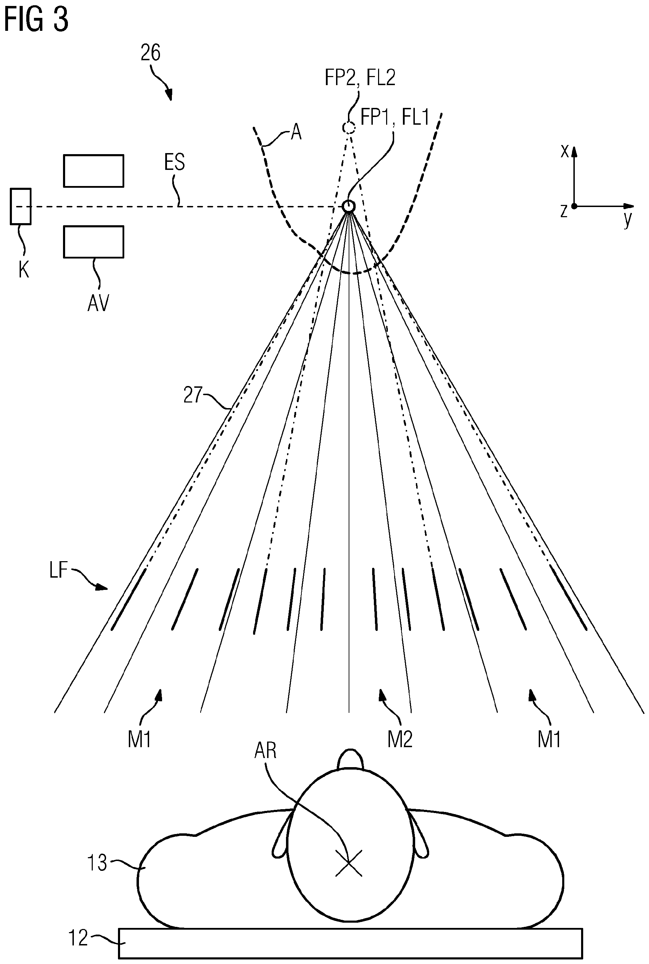

Die Fig. 3 zeigt eine Röntgenquelle 26 in einem ersten Betriebszustand und einen Lamellenformfilter LF mit einer ersten Fokuslinie FL1 und einer zweiten Fokuslinie FL2. Der Lamellenformfilter LF weist eine zweite Mehrzahl von zweiten Lamellen M2 auf, welche auf die zweite Fokuslinie FL2 fokussiert sind. Der zweite Fokuspunkt FP2 befindet sich auf der zweiten Fokuslinie FL2. Die zweite Fokuslinie FL2 ist parallel zu der ersten Fokuslinie FL1. Die erste Fokuslinie FL1 und die zweite Fokuslinie FL2 sind im Wesentlichen parallel zu der Rotationsachse AR.

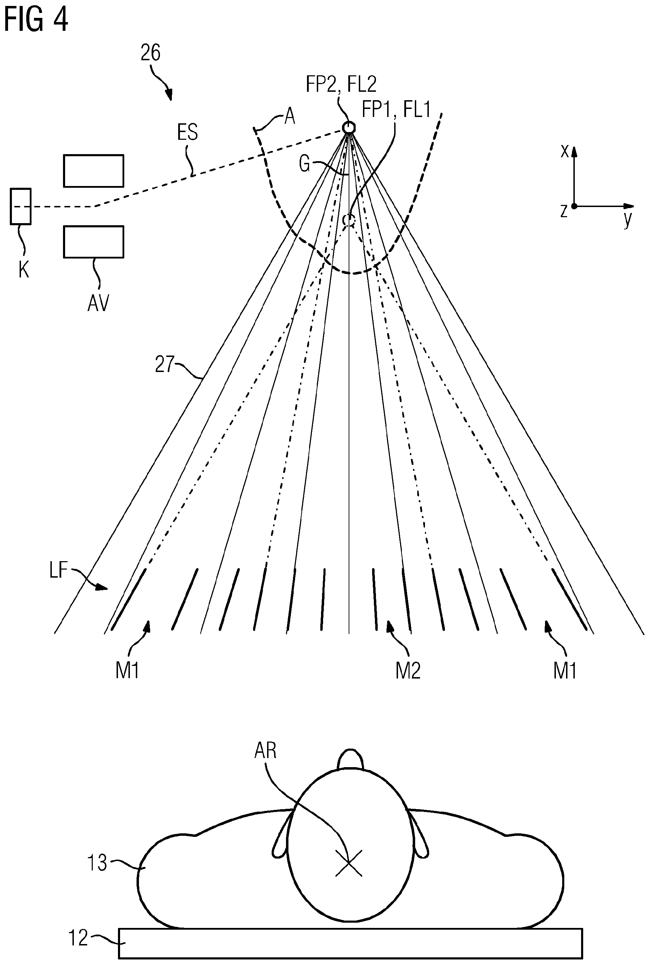

Die Fig. 4 zeigt die Röntgenquelle 26 und den Lamellenformfilter LF gemäß dem in der Fig. 3 gezeigten Ausführungsbeispiel, wobei die Röntgenquelle 26 in einem zweiten Betriebszustand ist.

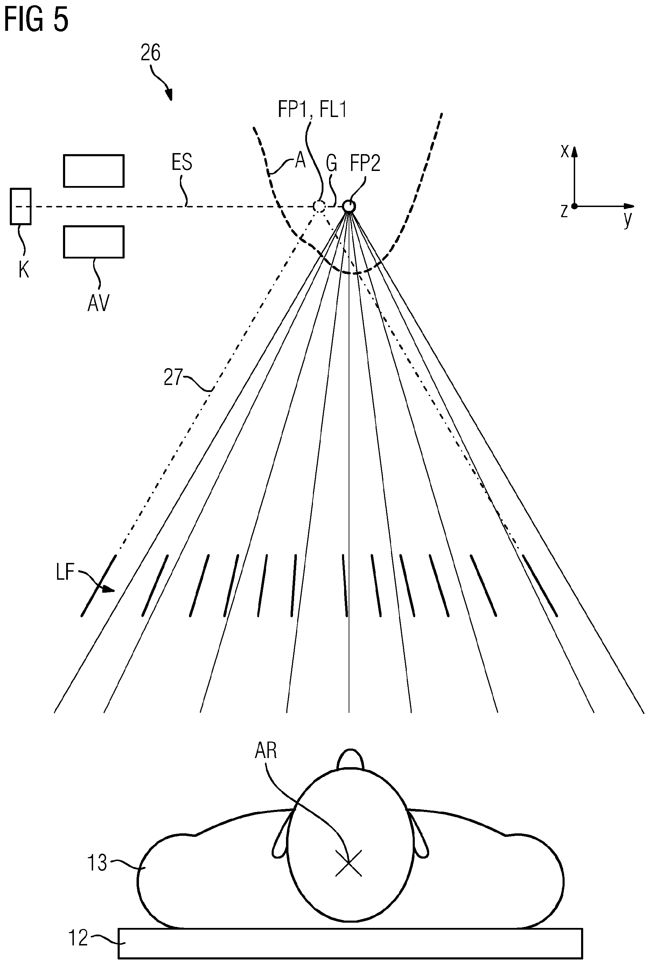

Die Fig. 5 zeigt eine Röntgenquelle 26 und einen Lamellenformfilter LF, wobei der erste Fokuspunkt FP1 und der zweite Fokuspunkt FP2 relativ zueinander entlang einer Breite des Fächerstrahls der Röntgenstrahlung 27 versetzt sind. Der erste Fokuspunkt FP1 und der zweite Fokuspunkt FP2 sind relativ zueinander in Bezug auf eine Umfangsrichtung y versetzt, welche im Wesentlichen senkrecht zu einer Ebene ist, in welcher sich die Rotationsachse AR befindet und in welcher sich ferner der erste Fokuspunkt FP1 oder der zweite Fokuspunkt FP2 befindet.

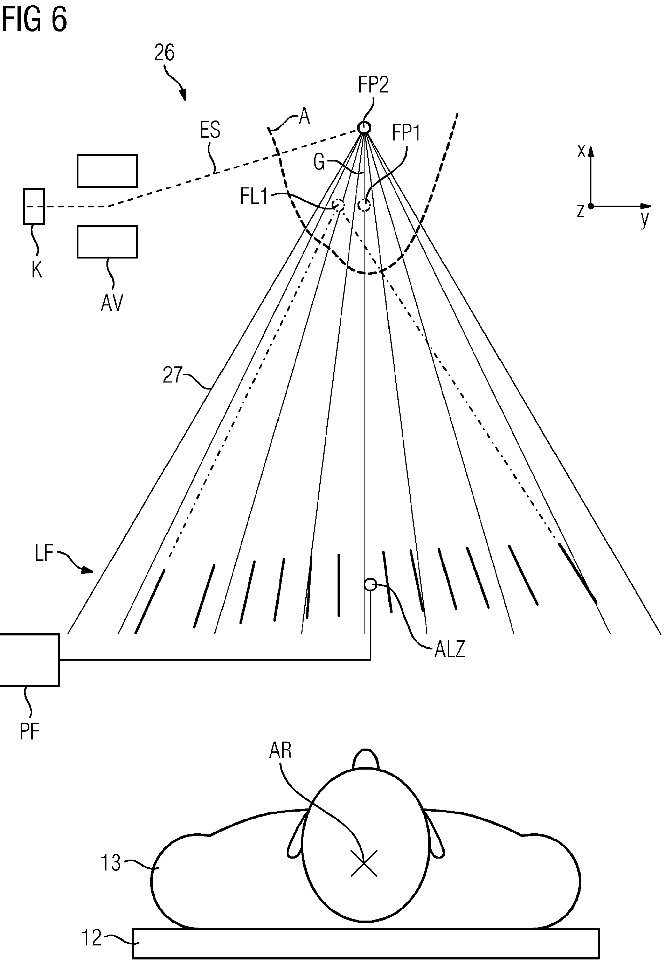

Die Fig. 6 zeigt eine Röntgenquelle 26 und einen Lamellenformfilter LF, wobei der Lamellenformfilter LF relativ zu der Röntgenquelle 26 positionierbar ist. Der Lamellenformfilter LF ist mittels der Positioniereinheit PF derart relativ zu der Röntgenquelle 26 positionierbar, dass durch eine Positionierung des Lamellenformfilters LF relativ zu der Röntgenquelle 26 eine Lage der ersten Fokuslinie FL1 relativ zu dem ersten Fokuspunkt FP1 geändert werden kann. Alternativ oder zusätzlich dazu sind die ersten Lamellen M1 des Lamellenformfilters LF mittels der Positioniereinheit PF derart relativ zueinander positionierbar, dass durch eine Positionierung der ersten Lamellen M1 des Lamellenformfilters LF relativ zueinander eine Lage der ersten Fokuslinie FL1 relativ zu dem ersten Fokuspunkt FP1 geändert werden kann.

Der Lammellenformfilter LF ist um die Schwenkachse ALZ schwenkbar gelagert, welche parallel zu der Rotationsachse AR ist. Die Positioniereinheit PF kann beispielsweise eine Schwenkbewegung des Lamellenformfilters LF um die Schwenkachse ALZ antreiben. Die Schwenkachse ALZ ist parallel zu der ersten Fokuslinie FL1.

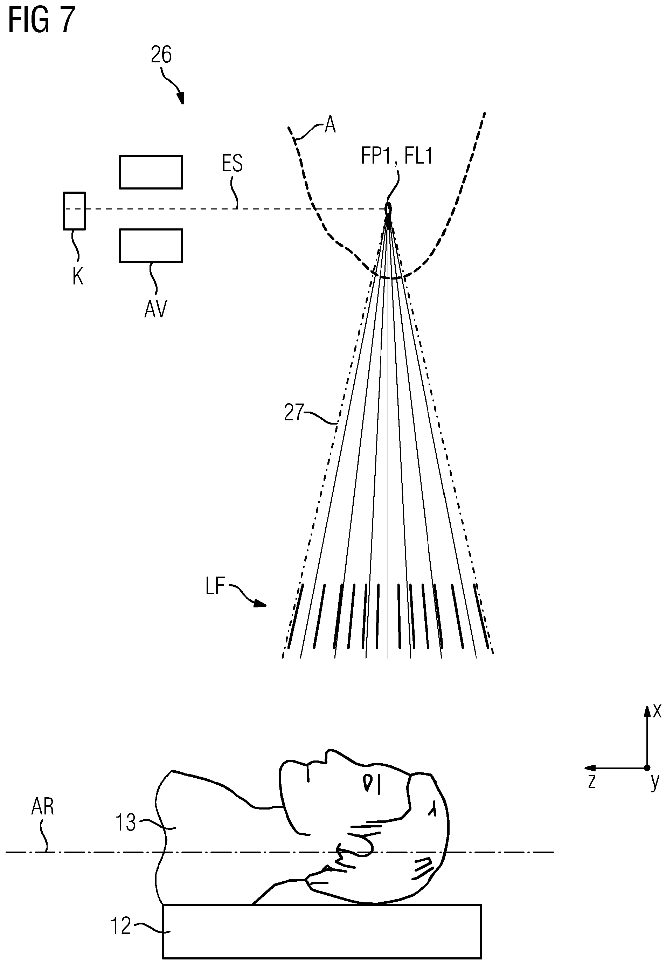

Die Fig. 7 zeigt eine Röntgenquelle 26 in einem ersten Betriebszustand und einen Lamellenformfilter LF, wobei die erste Fokuslinie FL1 in einer Ebene liegt, die senkrecht zu der Rotationsachse AR ist.

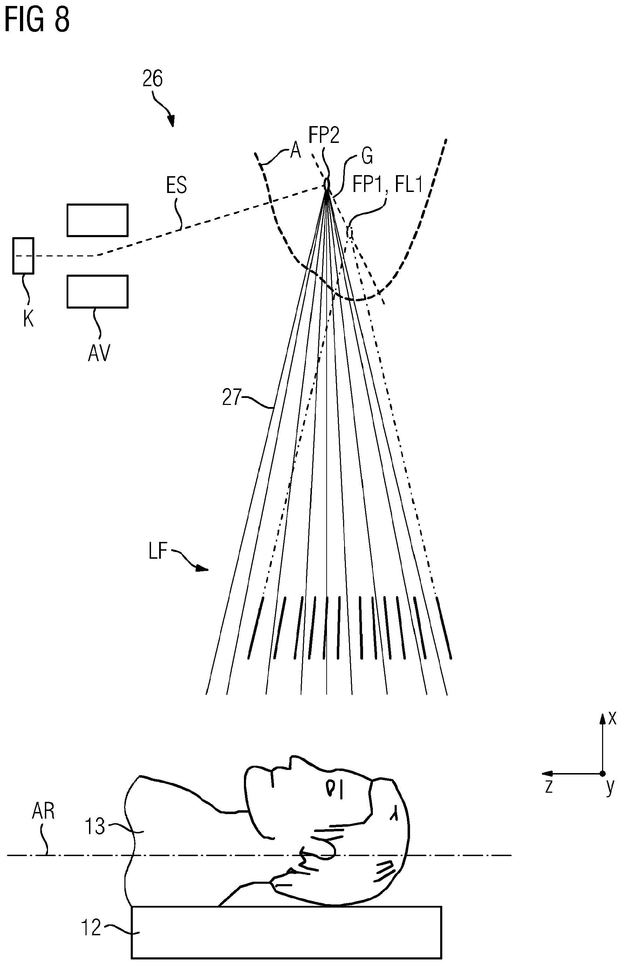

Die Fig. 8 zeigt die Röntgenquelle 26 und den Lamellenformfilter LF gemäß der in der Fig. 7 gezeigten Ausführungsform, wobei die Röntgenquelle 26 in einem zweiten Betriebszustand ist. Die erste Fokuslinie FL1 liegt in einer Ebene, die senkrecht zu der Rotationsachse AR ist. Der erste Fokuspunkt FP1 und der zweite Fokuspunkt FP2 sind relativ zueinander in Bezug auf die Radialrichtung x und in Bezug auf eine Axialrichtung z, welche parallel zu der Rotationsachse AR ist, versetzt. Die Rotationsachse AR, der erste Fokuspunkt FP1 und der zweite Fokuspunkt FP2 befinden sich in einer Ebene.

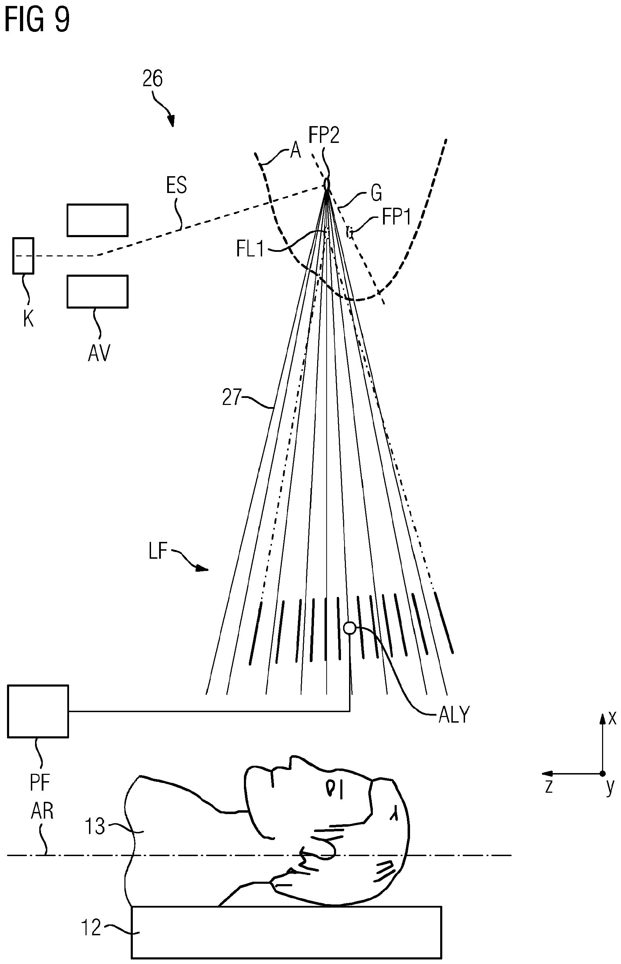

Die Fig. 9 zeigt die Röntgenquelle 26 und einen Lamellenformfilter LF, wobei die erste Fokuslinie FL1 in einer Ebene liegt, die senkrecht zu der Rotationsachse AR ist, wobei der Lamellenformfilter LF relativ zu der Röntgenquelle 26 positionierbar ist. Der Lamellenformfilter LF ist mittels der Positioniereinheit PF derart relativ zu der Röntgenquelle 26 positionierbar, dass durch eine Positionierung des Lamellenformfilters LF relativ zu der Röntgenquelle 26 eine Lage der ersten Fokuslinie FL1 relativ zu dem ersten Fokuspunkt FP1 geändert werden kann. Alternativ oder zusätzlich dazu sind die ersten Lamellen M1 des Lamellenformfilters LF mittels der Positioniereinheit PF derart relativ zueinander positionierbar, dass durch eine Positionierung der ersten Lamellen M1 des Lamellenformfilters LF relativ zueinander eine Lage der ersten Fokuslinie FL1 relativ zu dem ersten Fokuspunkt FP1 geändert werden kann.

Der Lammellenformfilter LF ist um die Schwenkachse ALY schwenkbar gelagert, welche parallel zu der Umfangsrichtung y ist und sich insbesondere in einer Ebene befindet, die senkrecht zu der Rotationsachse AR ist. Die Positioniereinheit PF kann beispielsweise eine Schwenkbewegung des Lamellenformfilters LF um die Schwenkachse ALY antreiben. Die Schwenkachse ALY ist parallel zu der ersten Fokuslinie FL1.

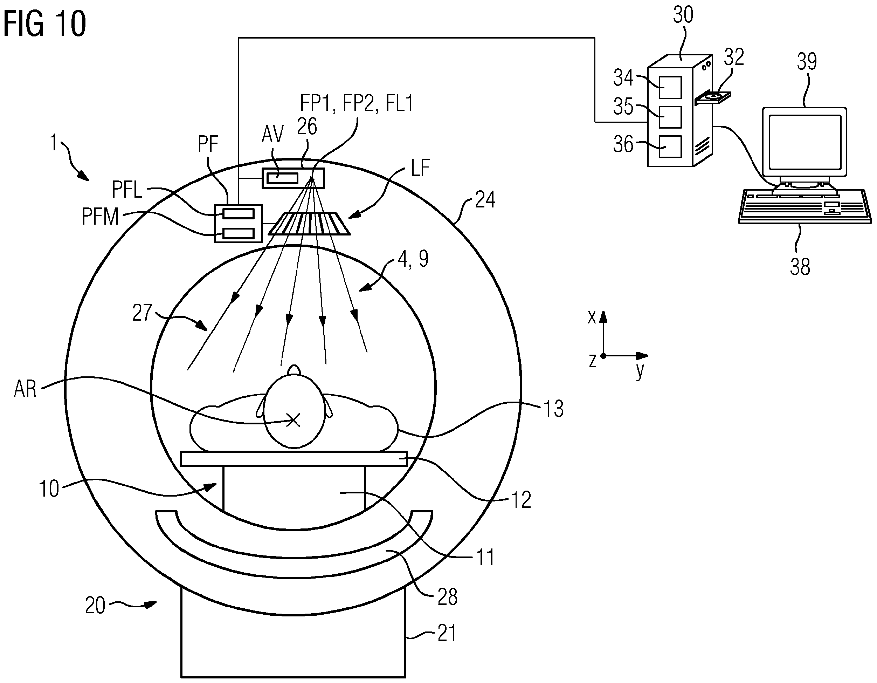

Die Fig. 10 zeigt ein Computertomographiegerät 1 mit einer Röntgenquelle 26 und einem Lamellenformfilter LF. Das Computertomographiegerät 1 weist die Gantry 20, die tunnelförmige Öffnung 9, die Patientenlagerungsvorrichtung 10 und die Steuerungsvorrichtung 30 auf. Die Gantry 20 weist den Tragrahmen 21 und den Rotor 24 auf. Das Computertomographiegerät 1 kann optional einen Kipprahmen aufweisen, der mittels einer Kipplagerungsvorrichtung an dem Tragrahmen 21 um eine Kippachse relativ zu dem Tragrahmen 21 kippbar angeordnet ist, wobei die Kippachse senkrecht zu der Rotationsachse AR ist und sich in einer horizontalen Ebene befindet.

Wenn das Computertomographiegerät 1 einen Kipprahmen aufweist, ist der Rotor 24 mittels einer Drehlagerungsvorrichtung an dem Kipprahmen um die Rotationsachse AR relativ zu dem Kipprahmen drehbar angeordnet. Ansonsten ist der Rotor 24 mittels einer Drehlagerungsvorrichtung an dem Tragrahmen 21 um die Rotationsachse AR relativ zu dem Kipprahmen 21 drehbar angeordnet.

In die tunnelförmige Öffnung 9 ist der Patient 13 einführbar. In der tunnelförmigen Öffnung 9 befindet sich der Akquisitionsbereich 4. In dem Akquisitionsbereich 4 ist ein abzubildender Bereich des Patienten 13 derart positionierbar, dass die Strahlung 27 von der Strahlungsquelle 26 zu dem abzubildenden Bereich gelangen kann und nach einer Wechselwirkung mit dem abzubildenden Bereich zu dem Strahlungsdetektor 28 gelangen kann. Die Patientenlagerungsvorrichtung 10 weist den Lagerungssockel 11 und die Lagerungsplatte 12 zur Lagerung des Patienten 13 auf. Die Lagerungsplatte 12 ist derart relativ zu dem Lagerungssockel 11 bewegbar an dem Lagerungssockel 11 angeordnet, dass die Lagerungsplatte 12 in einer Längsrichtung der Lagerungsplatte 12, insbesondere im Wesentlichen entlang der Rotationsachse AR, in den Akquisitionsbereich 4 einführbar ist.

Das Computertomographiegerät 1 ist zur Akquisition von Akquisitionsdaten basierend auf einer elektromagnetischen Strahlung 27 ausgebildet. Das Computertomographiegerät 1 weist eine Akquisitionseinheit auf. Die Akquisitionseinheit ist eine Projektionsdaten-Akquisitionseinheit mit der Strahlungsquelle 26, z. B. einer Röntgenquelle, und dem Detektor 28, z. B. einem Röntgendetektor, insbesondere einem energieauflösenden Röntgendetektor.

Die Strahlungsquelle 26 ist an dem Rotor 24 angeordnet und zur Emission einer Strahlung 27, z. B. einer Röntgenstrahlung, mit Strahlungsquanten 27 ausgebildet. Der Detektor 28 ist an dem Rotor 24 angeordnet und zur Detektion der Strahlungsquanten 27 ausgebildet. Die Strahlungsquanten 27 können von der Strahlungsquelle 26 zu dem abzubildenden Bereich des Patienten 13 gelangen und nach einer Wechselwirkung mit dem abzubildenden Bereich auf den Detektor 28 auftreffen. Auf diese Weise können mittels der Akquisitionseinheit Akquisitionsdaten des abzubildenden Bereichs in Form von Projektionsdaten erfasst werden.

Das Computertomographiegerät 1 weist eine Positioniereinheit PF auf. Die Positioniereinheit PF weist ein Lamellenformfilter-Positioniermodul PFL auf, welches zum Positionieren des Lamellenformfilters LF relativ zu der Röntgenquelle 26 ausgebildet ist. Die Positioniereinheit PF weist ferner ein Lamellen-Positioniermodul PFM auf, welches zum Positionieren der ersten Lamellen M1 des Lamellenformfilters LF relativ zueinander ausgebildet ist. Die Positioniereinheit PF ist somit zum Positionieren des Lamellenformfilters LF relativ zu der Röntgenquelle 26 und zum Positionieren der ersten Lamellen M1 des Lamellenformfilters LF relativ zueinander ausgebildet. Der Lamellenformfilter LF ist mittels des Lamellenformfilter-Positioniermoduls PFL mit dem Rotor 24 verbunden. Das Lamellenformfilter-Positioniermoduls PFL kann beispielsweise eine kardanische Aufhängung aufweisen.

Die Steuerungsvorrichtung 30 ist zum Empfangen der von der Akquisitionseinheit akquirierten Akquisitionsdaten ausgebildet. Die Steuerungsvorrichtung 30 ist zum Steuern des Computertomographiegeräts 1 ausgebildet. Die Steuerungsvorrichtung 30 weist die Datenverarbeitungseinheit 35, das computerlesbare Medium 32 und das Prozessorsystem 36 auf. Die Steuerungsvorrichtung 30, insbesondere die Datenverarbeitungseinheit 35, wird von einem Datenverarbeitungssystem, welches einen Computer aufweist, gebildet. Die Datenverarbeitungseinheit 35 ist insbesondere zum Steuern der Ablenkvorrichtung AV und/oder zum Steuern der Positioniereinheit PF ausgebildet.

Die Steuerungsvorrichtung 30 weist die Bildrekonstruktionseinrichtung 34 auf. Mittels der Bildrekonstruktionseinrichtung 34 kann basierend auf den Akquisitionsdaten ein medizinischer Bilddatensatz rekonstruiert werden. Mittels der Bildrekonstruktionseinrichtung 34 kann insbesondere ein medizinisches Bild basierend auf den ersten Projektionsdaten und den zweiten Projektionsdaten erzeugt werden.

Das Computertomographiegerät 1 weist eine Eingabevorrichtung 38 und eine Ausgabevorrichtung 39 auf, welche jeweils mit der Steuerungsvorrichtung 30 verbunden sind. Die Eingabevorrichtung 38 ist zum Eingeben von Steuerungs-Informationen, z. B. Bildrekonstruktionsparametern, Untersuchungsparametern oder ähnliches, ausgebildet. Die Ausgabevorrichtung 39 ist insbesondere zum Ausgeben von Steuerungs-Informationen, Bildern und/oder akustischen Signalen ausgebildet.

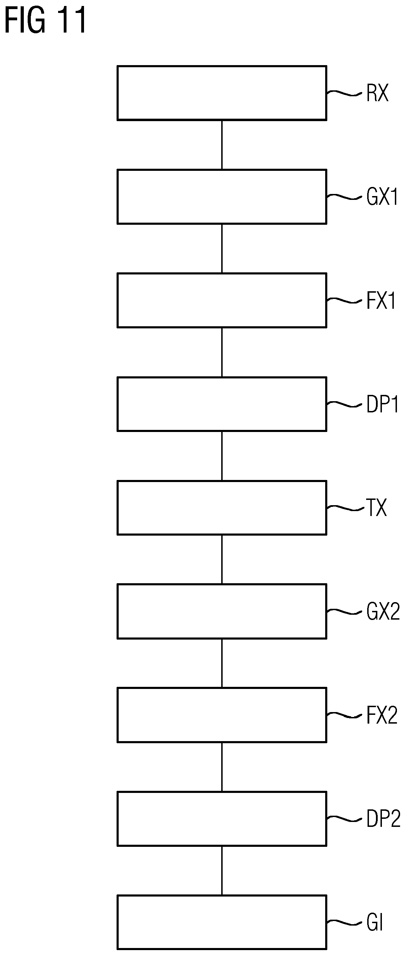

Die Fig. 11 zeigt ein Ablaufdiagramm eines Verfahrens zum Erzeugen eines medizinischen Bildes, wobei das Verfahren die folgenden Schritte umfasst:
- Drehen RX einer Röntgenquelle 26 und eines Röntgendetektors 28, der mit der Röntgenquelle 26 zusammenwirkt, um eine Rotationsachse AR, die sich zwischen der Röntgen-quelle 26 und dem Röntgendetektor 28 befindet,
- Erzeugen GX1 einer Röntgenstrahlung 27 mittels einer Röntgenquelle 26 an einem ersten Fokuspunkt FP1, während die Röntgenquelle 26 in einem ersten Betriebszustand der Röntgenquelle 26 ist,
- Filtern FX1 der Röntgenstrahlung 27, die an dem ersten Fokuspunkt FP1 erzeugt wurde, mittels eines Lamellenformfilters LF, der zwischen der Röntgenquelle 26 und der Rotationsachse AR angeordnet ist, wobei der Lamellenformfilter LF eine erste Mehrzahl von ersten Lamellen M1 aufweist, welche auf eine erste Fokuslinie FL1 fokussiert sind,
- Erfassen DP1 von ersten Projektionsdaten mittels des Röntgendetektors 28 basierend auf der Röntgenstrahlung 27, welche an dem ersten Fokuspunkt FP1 erzeugt wurde und mittels des Lamellenformfilters LF gefiltert wurde,
- Übergang TX von dem ersten Betriebszustand der Röntgenquelle 26 in einen zweiten Betriebszustand der Röntgenquelle 26,
- Erzeugen GX2 der Röntgenstrahlung 27 mittels der Röntgenquelle 26 an einem zweiten Fokuspunkt FP2, während die Röntgenquelle 26 in dem zweiten Betriebszustand der Röntgenquelle 26 ist,
- Filtern FX2 der Röntgenstrahlung 27, die an dem zweiten Fokuspunkt FP2 erzeugt wurde, mittels des Lamellenformfilters LF, der zwischen der Röntgenquelle 26 und der Rotationsachse AR angeordnet ist, wobei der Lamellenformfilter LF eine erste Mehrzahl von ersten Lamellen M1 aufweist, welche auf eine erste Fokuslinie FL1 fokussiert sind,
- Erfassen DP2 von zweiten Projektionsdaten mittels des Röntgendetektors 28 basierend auf der Röntgenstrahlung 27, welche an dem zweiten Fokuspunkt FP2 erzeugt wurde und mittels des Lamellenformfilters LF gefiltert wurde,
- Erzeugen GI eines medizinischen Bildes basierend auf den ersten Projektionsdaten und den zweiten Projektionsdaten.

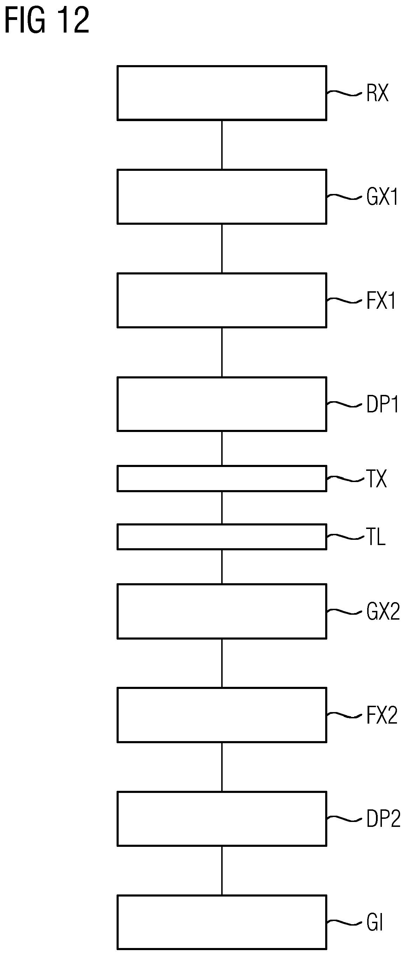

Die Fig. 12 zeigt ein Ablaufdiagramm eines Verfahrens zum Erzeugen eines medizinischen Bildes, wobei das Verfahren ferner den folgenden Schritt umfasst:
- Übergang TL von einem ersten Betriebszustand des Lamellenformfilters LF in einen zweiten Betriebszustand des Lamellenformfilters LF derart, dass sich dabei die Lage der ersten Fokuslinie relativ zu dem ersten Fokuspunkt FP1 ändert,
- wobei die Röntgenstrahlung 27 mittels der Röntgenquelle 26 an dem ersten Fokuspunkt FP1 erzeugt wird, während die Röntgenquelle 26 in dem ersten Betriebszustand der Röntgenquelle 26 ist und der Lamellenformfilter LF in dem ersten Betriebszustand des Lamellenformfilters LF ist,
- wobei die Röntgenstrahlung 27 mittels der Röntgenquelle 26 an dem zweiten Fokuspunkt FP2 erzeugt wird, während die Röntgenquelle 26 in dem zweiten Betriebszustand der Röntgenquelle 26 ist und der Lamellenformfilter LF in dem zweiten Betriebszustand des Lamellenformfilters LF ist.

Insbesondere kann der Übergang TX von dem ersten Betriebszustand der Röntgenquelle 26 in den zweiten Betriebszustand der Röntgenquelle 26 und/oder der Übergang TL von dem ersten Betriebszustand des Lamellenformfilters LF in den zweiten Betriebszustand des Lamellenformfilters LF in Abhängigkeit von einem Drehwinkel der Drehung der Röntgenquelle 26 und des Röntgendetektors 28 um die Rotationsachse AR erfolgen.

## Patentansprüche

1. Computertomographiegerät (1), aufweisend:
- eine Röntgenquelle (26) und einen Röntgendetektor (28), der mit der Röntgenquelle (26) zusammenwirkt, wobei die Röntgenquelle (26) und der Röntgendetektor (28) um eine Rotationsachse (AR), die sich zwischen der Röntgenquelle (26) und dem Röntgendetektor (28) befindet, drehbar angeordnet sind,
- einen Lamellenformfilter (LF), der zwischen der Röntgenquelle (26) und der Rotationsachse (AR) angeordnet ist, wobei der Lamellenformfilter (LF) eine erste Mehrzahl von ersten Lamellen (M1) aufweist, welche auf eine erste Fokuslinie (FL1) fokussiert sind,
- wobei die Röntgenquelle (26) dazu ausgebildet ist, Röntgenstrahlung (27) in einem ersten Betriebszustand der Röntgenquelle (26) an einem ersten Fokuspunkt (FP1) zu erzeugen und in einem zweiten Betriebszustand der Röntgenquelle (26) an einem zweiten Fokuspunkt (FP2) zu erzeugen.

2. Computertomographiegerät (1) nach Anspruch 1,
- wobei der erste Fokuspunkt (FP1) und der zweite Fokuspunkt (FP2) relativ zueinander zumindest in Bezug auf eine Axialrichtung (z) versetzt sind, welche parallel zu der Rotationsachse (AR) ist.

3. Computertomographiegerät (1) nach Anspruch 1 oder 2,
- wobei der erste Fokuspunkt (FP1) und der zweite Fokuspunkt (FP2) relativ zueinander zumindest in Bezug auf eine Radialrichtung (x) versetzt sind, welche die Rotationsachse (AR) schneidet und welche senkrecht zu der Rotationsachse (AR) ist.

4. Computertomographiegerät (1) nach einem der Ansprüche 1 bis 3,
- wobei der erste Fokuspunkt (FP1) und der zweite Fokuspunkt (FP2) relativ zueinander zumindest in Bezug auf eine Umfangsrichtung (y) versetzt sind, welche im Wesentlichen senkrecht zu einer Ebene ist, in welcher sich die Rotationsachse (AR) befindet und in welcher sich ferner der erste Fokuspunkt (FP1) oder der zweite Fokuspunkt (FP2) befindet.

5. Computertomographiegerät (1) nach einem der Ansprüche 1 bis 3,
- wobei sich die Rotationsachse (AR), der erste Fokuspunkt (FP1) und der zweite Fokuspunkt (FP2) in einer Ebene befinden.

6. Computertomographiegerät (1) nach einem der Ansprüche 1 bis 5,
- wobei sich der erste Fokuspunkt (FP1) auf der ersten Fokuslinie (FL1) befindet,
- wobei sich der zweite Fokuspunkt (FP2) nicht auf der ersten Fokuslinie (FL1) befindet.

7. Computertomographiegerät (1) nach einem der Ansprüche 1 bis 6,
- wobei der Lamellenformfilter (LF) eine zweite Mehrzahl von zweiten Lamellen (M2) aufweist, welche auf eine zweite Fokuslinie (FL2) fokussiert sind,
- wobei sich der zweite Fokuspunkt (FP2) auf der zweiten Fokuslinie (FL2) befindet.

8. Computertomographiegerät (1) nach Anspruch 7,
- wobei die zweite Fokuslinie (FL2) parallel zu der ersten Fokuslinie (FL1) ist.

9. Computertomographiegerät (1) nach einem der Ansprüche 1 bis 8,
- wobei die erste Fokuslinie (FL1) und/oder die zweite Fokuslinie (FL2) im Wesentlichen parallel zu der Rotationsachse (AR) ist.

10. Computertomographiegerät (1) nach einem der Ansprüche 1 bis 8,
- wobei die erste Fokuslinie (FL1) und/oder die zweite Fokuslinie (FL2) in einer Ebene liegt, die senkrecht zu der Rotationsachse (AR) ist.

11. Computertomographiegerät (1) nach einem der Ansprüche 1 bis 10,
- wobei die Röntgenquelle (26) eine Kathode (K), eine Anode (A) und eine Ablenkvorrichtung (AV) aufweist,
- wobei die Ablenkvorrichtung (AV) dazu ausgebildet ist, eine Ablenkung eines Elektronenstrahls (ES), der von der Kathode (K) ausgeht, derart einzustellen, dass der Elektronenstrahl (ES) in dem ersten Betriebszustand der Röntgenquelle (26) in dem ersten Fokuspunkt (FP1) auf die Anode (A) trifft und in dem zweiten Betriebszustand der Röntgenquelle (26) in dem zweiten Fokuspunkt (FP2) auf die Anode (A) trifft.

12. Computertomographiegerät (1) nach einem der Ansprüche 1 bis 11, ferner aufweisend:
- eine Positioniereinheit (PF),
- wobei der Lamellenformfilter (LF) mittels der Positioniereinheit (PF) derart relativ zu der Röntgenquelle (26) positionierbar ist, dass durch eine Positionierung des Lamellenformfilters (LF) relativ zu der Röntgenquelle (26) eine Lage der ersten Fokuslinie (FL1) relativ zu dem ersten Fokuspunkt (FP1) geändert werden kann, und/oder
- wobei die ersten Lamellen (M1) des Lamellenformfilters (LF) mittels der Positioniereinheit (PF) derart relativ zueinander positionierbar sind, dass durch eine Positionierung der ersten Lamellen (M1) des Lamellenformfilters (LF) relativ zueinander eine Lage der ersten Fokuslinie (FL1) relativ zu dem ersten Fokuspunkt (FP1) geändert werden kann.

13. Verfahren zum Erzeugen eines medizinischen Bildes, wobei das Verfahren die folgenden Schritte umfasst:
- Drehen (RX) einer Röntgenquelle (26) und eines Röntgendetektors (28), der mit der Röntgenquelle (26) zusammenwirkt, um eine Rotationsachse (AR), die sich zwischen der Röntgenquelle (26) und dem Röntgendetektor (28) befindet,
- Erzeugen (GX1) einer Röntgenstrahlung (27) mittels einer Röntgenquelle (26) an einem ersten Fokuspunkt (FP1), während die Röntgenquelle (26) in einem ersten Betriebszustand der Röntgenquelle (26) ist,
- Filtern (FX1) der Röntgenstrahlung (27), die an dem ersten Fokuspunkt (FP1) erzeugt wurde, mittels eines Lamellenformfilters (LF), der zwischen der Röntgenquelle (26) und der Rotationsachse (AR) angeordnet ist, wobei der Lamellenformfilter (LF) eine erste Mehrzahl von ersten Lamellen (M1) aufweist, welche auf eine erste Fokuslinie (FL1) fokussiert sind,
- Erfassen (DP1) von ersten Projektionsdaten mittels des Röntgendetektors (28) basierend auf der Röntgenstrahlung (27), welche an dem ersten Fokuspunkt (FP1) erzeugt wurde und mittels des Lamellenformfilters (LF) gefiltert wurde,
- Übergang (TX) von dem ersten Betriebszustand der Röntgenquelle (26) in einen zweiten Betriebszustand der Röntgenquelle (26),
- Erzeugen (GX2) der Röntgenstrahlung (27) mittels der Röntgenquelle (26) an einem zweiten Fokuspunkt (FP2), während die Röntgenquelle (26) in dem zweiten Betriebszustand der Röntgenquelle (26) ist,
- Filtern (FX2) der Röntgenstrahlung (27), die an dem zweiten Fokuspunkt (FP2) erzeugt wurde, mittels des Lamellenformfilters (LF), der zwischen der Röntgenquelle (26) und der Rotationsachse (AR) angeordnet ist, wobei der Lamellenformfilter (LF) eine erste Mehrzahl von ersten Lamellen (M1) aufweist, welche auf eine erste Fokuslinie (FL1) fokussiert sind,
- Erfassen (DP2) von zweiten Projektionsdaten mittels des Röntgendetektors (28) basierend auf der Röntgenstrahlung (27), welche an dem zweiten Fokuspunkt (FP2) erzeugt wurde und mittels des Lamellenformfilters (LF) gefiltert wurde,
- Erzeugen (GI) eines medizinischen Bildes basierend auf den ersten Projektionsdaten und den zweiten Projektionsdaten.

14. Verfahren nach Anspruch 13, wobei das Verfahren ferner den folgenden Schritt umfasst:
- Übergang (TL) von einem ersten Betriebszustand des Lamellenformfilters (LF) in einen zweiten Betriebszustand des Lamellenformfilters (LF) derart, dass sich dabei die Lage der ersten Fokuslinie (FL1) relativ zu dem ersten Fokuspunkt (FP1) ändert,
- wobei die Röntgenstrahlung (27) mittels der Röntgenquelle (26) an dem ersten Fokuspunkt (FP1) erzeugt wird, während die Röntgenquelle (26) in dem ersten Betriebszustand der Röntgenquelle (26) ist und der Lamellenformfilter (LF) in dem ersten Betriebszustand des Lamellenformfilters (LF) ist,
- wobei die Röntgenstrahlung (27) mittels der Röntgenquelle (26) an dem zweiten Fokuspunkt (FP2) erzeugt wird, während die Röntgenquelle (26) in dem zweiten Betriebszustand der Röntgenquelle (26) ist und der Lamellenformfilter (LF) in dem zweiten Betriebszustand des Lamellenformfilters (LF) ist.

15. Verfahren nach Anspruch 13 oder 14, wobei der Übergang (TX) von dem ersten Betriebszustand der Röntgenquelle (26) in den zweiten Betriebszustand der Röntgenquelle (26) und/oder der Übergang (TL) von dem ersten Betriebszustand des Lamellenformfilters (LF) in den zweiten Betriebszustand des Lamellenformfilters (LF) in Abhängigkeit von einem Drehwinkel der Drehung der Röntgenquelle (26) und des Röntgendetektors (28) um die Rotationsachse (AR) erfolgt.
